# FASCICULE DE BREVET EUROPEEN

(11) **EP 2 416 836 B1**
(45) Date de publication et mention de la délivrance du brevet: **19.06.2013**
(21) Numéro de dépôt: 10711890.3
(22) Date de dépôt: 30.03.2010
(51) Int. Cl.: A61M 1/16

(54) **INSERT POUR RECIPIENT CONTENANT DES PRODUITS SOLIDES A DISSOUDRE**
EINLAGE FÜR BEHÄLTER ENTHALTEND FESTE, ZU AUFLÖSENDE, STOFFE
INSERT FOR A RECIPIENT CONTAINING SOLID PRODUCTS TO BE DISSOLVED

(30) Priorité: 08.04.2009 FR 0952299
(43) Date de publication de la demande: 15.02.2012
(73) Titulaire: Fresenius Medical Care Deutschland GmbH, 61352 Bad Homburg (DE)
(72) Inventeur: DUMONT D'AYOT, François, 69005 Lyon (FR); GASTAUER, Paul, Hong Kong (CN); LAFFAY, Philippe, 69110 Sainte Foy-Lès-Lyon (FR)
(74) Mandataire: Vièl, Frédérique
(86) Numéro de dépôt international: PCT/EP2010/054252
(87) Numéro de publication internationale: WO 2010/115787

(56) Documents cités:
- EP-A- 2 005 934
- WO-A-2006/042016
- DE-A1- 19 737 747

## Description

L'invention concerne un insert pour un récipient contenant un produit concentré sous forme solide ou liquide, comprenant une partie principale formée d'un fond se prolongeant sur le bord par une paroi perpendiculaire au fond, une ouverture étant réalisée dans le fond. L'invention concerne également un récipient muni d'un insert conforme à l'invention.

Par récipient on entend aussi bien une poche souple, qu'une cartouche, un flacon ou tout autre contenant apte à recevoir un concentré liquide ou solide et de l'eau ou un autre liquide pour solubiliser ou diluer ce concentré.

Il est courant en hémodialyse d'utiliser des solutions de dialyse qui sont réalisées directement avant la dialyse ou même pendant celle-ci. Pour cela, les machines d'hémodialyse sont équipées de dispositifs pour réaliser ces solutions. Le produit pharmaceutique se trouve sous forme de concentré, en général des poudres ou des granulés contenus dans des poches ou des cartouches. Ces poches ou cartouches sont connectées à la machine de dialyse qui introduit l'eau dans le récipient et aspire la solution ainsi réalisée.

Les raccords se trouvent dans certains cas dans le haut du récipient, dans d'autres cas dans le fond du récipient. Un exemple d'une poche munie de raccords dans le fond de la poche est connu de DE 101 52 105 A1. Les raccords sont situés approximativement au premier et au deuxième tiers du fond de la poche, en saillant à l'intérieur de la poche. Cette solution a pour inconvénient qu'il se forme des zones qui ne sont pas balayées par le flux d'eau de sorte qu'à la fin de la procédure de solubilisation, des restes de sels s'accumulent dans le fond sans qu'ils soient entraînés et solubilisés par l'eau introduite dans le récipient.

On connaît de EP 1 084 691 A2 un insert dont la tête saille également dans le récipient. Cet insert est constitué d'une partie principale en forme de vaisseau. Cette partie principale est constituée d'un fond plat dont le pourtour se prolonge par une paroi perpendiculaire dirigée vers la face externe du fond. Cette paroi en forme de vaisseau sert à souder la poche. Une ouverture est pratiquée au centre du fond, laquelle ouverture se prolonge sur la face externe par une tubulure. Des renforts transversaux et longitudinaux sont prévus sur la face externe du fond. La tubulure se poursuit sur la face interne du fond pour aboutir dans un élément de tête. Le canal se partage dans l'élément de tête en deux parties horizontales qui débouchent dans la poche bien au-dessus du fond. Cet insert présente donc les mêmes inconvénients que ceux évoqués précédemment.

On connait de EP 2 005 934 A2 un insert conforme au préambule de la revendication principale. Cet insert est fixé sur une poche contenant une solution médicale prête à l'emploi. Il présente un fond plat. Une première ouverture est pratiquée dans ce fond plat. Cette ouverture se prolonge sur la face interne de l'insert par une tubulure. De l'autre côté du fond plat, on trouve une deuxième ouverture prolongée sur la face externe de l'insert par une deuxième tubulure. La première ouverture est fermée hermétiquement par un bouchon pouvant être transpercé par l'aiguille d'un dispositif classique de perfusion. Par la seconde ouverture, il est possible d'une part d'introduire un composant « mineur » tel que de l'insuline et d'autre part de prélever la solution par une autre ligne de perfusion. Dans ce document, cette deuxième ouverture se trouve donc au raz du fond plat. Cependant, du fait de ce fond plat, cet insert s'il était utiliser pour des récipients contenant des produis solides, ne fonctionnerait pas de façon satisfaisante. En effet, du produit solide risquerait de s'accumuler sur le fond plat, notamment entre les deux ouvertures, derrière la tubulure interne ou dans le coin à l'extrémité du fond, du côté de la deuxième ouverture. De plus, les nervures de renfort réalisées sur la paroi perpendiculaire au fond constituent autant de recoins susceptibles de retenir le produit solide.

L'objectif de l'invention est donc de développer un insert qui permette de vider entièrement le récipient.

Cet objectif est atteint conformément à l'invention du fait que la face du fond destinée à être située dans le récipient après montage, face dite interne, est concave au niveau de l'ouverture de sorte que celle-ci se trouve au fond d'un creux. On évite ainsi qu'il se forme des recoins dans lesquels le liquide ne circule pas, ou pas assez. Le produit solide ne peut pas s'accumuler ailleurs que dans le creux de sorte qu'il est toujours soumis au courant du liquide.

Pour permettre une procédure continue, il est préférable de prévoir une deuxième ouverture dans le fond. La face interne du fond au niveau de cette deuxième ouverture est de préférence concave de sorte que la deuxième ouverture se trouve elle aussi au fond d'un creux.

Dans un mode de réalisation privilégié de l'invention, la face interne du fond forme une vague de sorte que chaque ouverture se trouve dans un creux séparé de l'autre par la crête de la vague.

Il est préférable que la paroi s'étende du côté de la face interne du fond et/ou du côté de la face opposée, dite face externe. Dans le cas du mode de réalisation privilégié en vague, le bord peut affleurer la face externe du fond au niveau des creux de la vague et affleurer la face interne au niveau de la crête de la vague en s'étendant vers la face interne au niveau des creux et vers la face externe au niveau de la crête.

Conformément à l'invention, des renforts solidaires des parois sont prévus sur l'une au moins des faces du fond, de préférence sur la face externe. Cela permet de fabriquer un insert creux très léger tout en étant pratiquement indéformable.

Afin de permettre le raccord du récipient muni de l'insert sur une machine d'hémodialyse, il est préférable que la ou l'une au moins des ouvertures se prolonge du côté de la face externe par une tubulure.

Dans une deuxième variante de réalisation de l'invention, un renfort est fixé sur la face externe du fond se prolongeant jusqu'aux deux tubulures, le renfort étant de préférence muni de moyens de blocage destinés à coopérer avec des moyens correspondants sur la machine de prélèvement et/ou de traverses perpendiculaires au renfort.

Afin de faciliter le soudage de la poche constituant le récipient sur l'insert, il est préférable que la partie principale ait la forme d'un vaisseau dont chaque pointe se prolonge de préférence par une languette. En général, le récipient est constitué par une poche réalisée en soudant deux bandes sur trois côtés, le quatrième côté étant soudé sur l'insert. Ainsi, les soudures des bords latéraux se finissent en interposant les languettes et chaque bord inférieur (quatrième bord) est soudé d'un côté du vaisseau, d'une languette à l'autre.

L'invention est présentée plus en détail ci-dessous à l'aide des figures qui montrent :
- Figure 1 :: Vue en perspective du côté de la face interne d'un insert conforme à l'invention ;
- Figure 2 :: Vue en perspective du côté de la face externe de l'insert de la figure 1 ;
- Figure 3 :: Vue en coupe longitudinale de l'insert de la figure 1 ;
- Figure 4 :: Vue en coupe transversale de l'insert de la figure 1 ;
- Figure 5 :: Vue de dessus de l'insert de la figure 1 ;
- Figure 6 :: Vue de dessous de l'insert de la figure 1 ;
- Figure 7 :: Vue en perspective d'un second mode de réalisation de l'insert conforme à l'invention, l'insert étant monté dans la machine d'hémodialyse.

L'insert selon l'invention est destiné à une poche, une cartouche ou tout autre récipient adapté contenant un produit sous forme de poudre ou de granulé devant être mis en solution par une machine d'hémodialyse. Il peut également s'agir d'un liquide devant être dilué. Le récipient fini est donc constitué d'un contenant fermé par l'insert de l'invention. Dans la pratique, l'insert peut être utilisé dans toute autre machine de mise en solution : bien qu'il soit toujours fait référence par la suite à une machine d'hémodialyse, il faut comprendre que l'invention peut être utilisée pour tout autre type de machine de mise en solution.

Pour permettre la mise en solution, l'insert comporte au moins une arrivée d'eau qui peut servir également de sortie pour le liquide. De préférence, l'insert comporte cependant une arrivée d'eau et une sortie distincte pour la solution réalisée afin de permettre une procédure continue. Les exemples de réalisation présentés concernent des inserts munis de deux ouvertures. La description ci-dessous peut cependant tout à fait être adaptée à un insert n'ayant qu'une seule ouverture comme cela sera montré plus loin.

L'insert conforme à l'invention est constitué d'un corps principal (1) formé d'un fond (2) se prolongeant sur son pourtour par une paroi (3). La paroi (3) sert essentiellement à souder le contenant, notamment la poche ou la cartouche. Une fois mis en place sur un contenant, l'insert présente donc une face interne et une face externe. Ces références spatiales sont conservées par analogie pour l'insert.

Deux trous (4, 5) sont réalisés dans le fond (2). Chaque trou (4, 5) se prolonge sur la face externe du fond (2) par une tubulure (10, 11). Ces tubulures sont destinées à raccorder l'insert à une machine d'hémodialyse. Les dimensions et le positionnement relatif des tubulures sont choisis en fonction du type de machine pour laquelle l'insert est destiné. Pour une machine de type Fresenius 5008 par exemple, l'écartement entre les axes des deux tubulures est de 72 mm, le diamètre des tubulures de 12,5 mm et leur hauteur de 9 mm. Un filtre poreux est introduit dans les ouvertures ou les tubulures pour éviter que le produit solide ne quitte le récipient. Ces filtres sont par exemple réalisés en polyéthylène fritté ou en PE micro-injecté.

Contrairement à l'état de la technique, le fond n'est pas plan, mais a la forme d'une vague. Le premier trou (4) se trouve dans un premier creux (6), le deuxième (5) dans un deuxième creux (7) de la vague, ces deux creux (6, 7) étant séparés par la crête (8) de la vague. Considéré du côté de la face interne, le fond présente donc une concavité au niveau de chaque trou (4, 5). En position d'utilisation dans la machine d'hémodialyse, les trous (4, 5) constituent les points les plus bas de la face interne du fond (2) de l'insert. Le produit solide tend donc à retomber dans les creux prés des ouvertures et se trouve ainsi toujours dans le flux de liquide, même lorsqu'il ne reste pratiquement plus de produit solide.

Afin de faciliter la soudure d'une poche sur l'insert, celui-ci a la forme d'un vaisseau, la crête de la vague étant située transversalement à ce vaisseau. Les extrémités du vaisseau sont munies de languettes (15). La poche est réalisée par exemple à l'aide de deux bandes soudées ensemble sur trois de leurs côtés, le quatrième étant soudé sur l'une des faces de la paroi (3) en vaisseau, d'une languette (15) à l'autre. Ainsi, le fond de la poche est réalisé par l'insert en forme de vaisseau, les bords latéraux de la poche partant directement de l'insert sans former de recoins susceptibles d'être hors du flux d'eau. Rien n'empêche que les parois latérales de la poche partant de l'insert s'éloignent en s'écartant ou en se rapprochant l'une de l'autre de sorte que la poche vue à plat a la forme d'un trapèze.

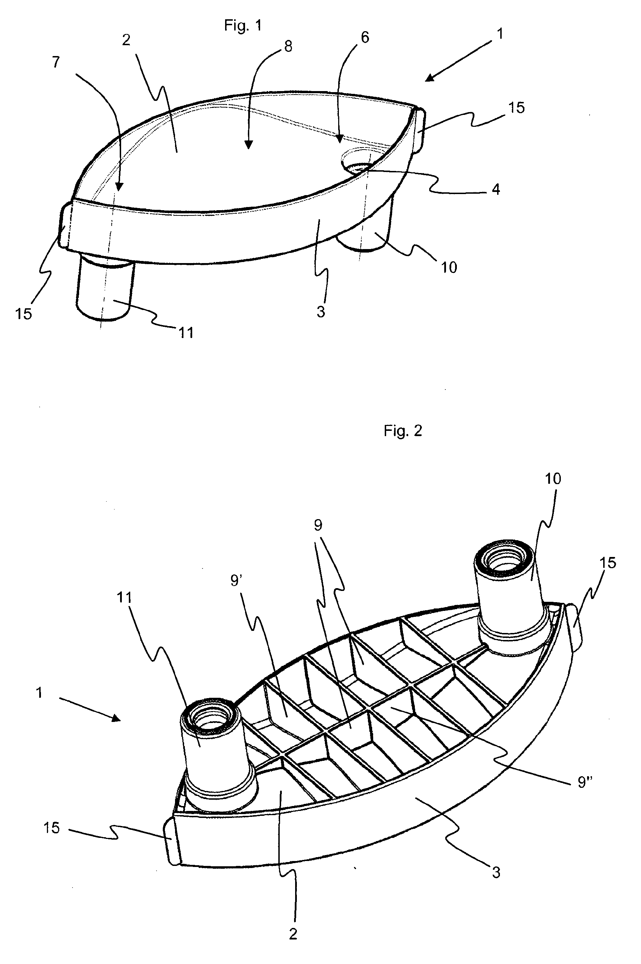

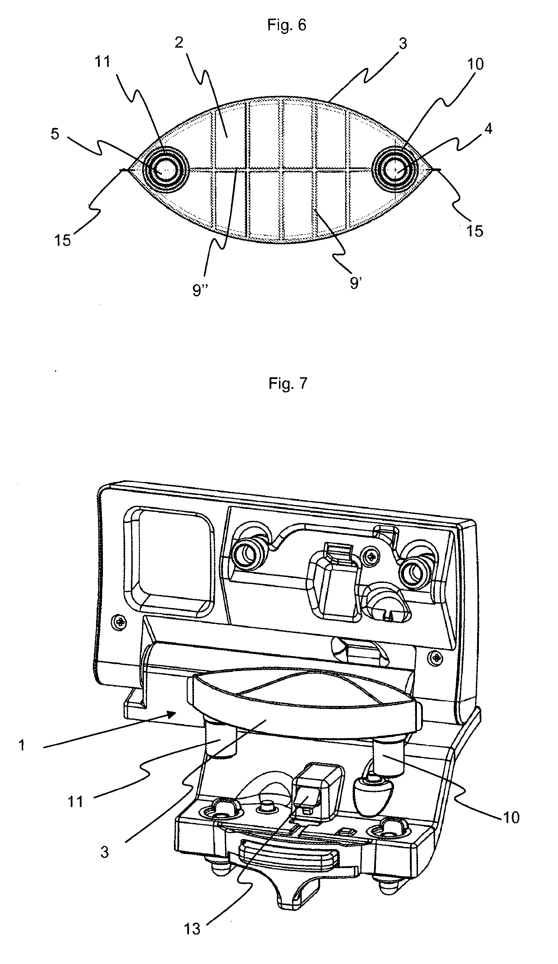

Dans l'exemple présenté aux figures 1 à 6, la paroi (3) s'étend en partie au-dessus et en partie au-dessous du fond (2). Au niveau des creux (6, 7) de la vague, c'est-à-dire des extrémités du vaisseau, la paroi s'étend du côté de la face interne du fond (2) tout en affleurant à la face externe du fond. Par contre, au niveau de la crête de la vague (8), elle s'étend du côté de la face externe du fond (2) tout en affleurant à la face interne. Il serait cependant tout à fait possible que la paroi ne s'étende que d'un côté du fond, côté interne ou côté externe, ou même qu'elle s'étende des deux côtés en dépassant des deux faces du fond.

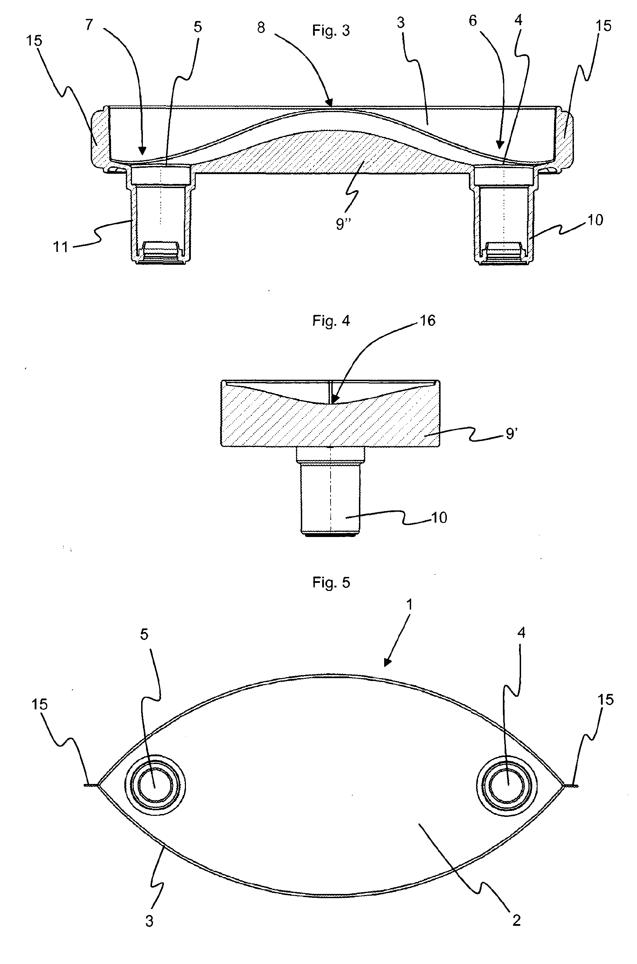

Afin d'assurer une meilleure retombée des particules solides dans les creux (6, 7) près des ouvertures (4, 5), il est prévu que la section transversale de la vague ne soit pas plane, mais qu'elle présente un creux (16), comme le montre la figure 4.

Dans l'exemple des figures 1 à 6, la face externe du corps principal (1) est creuse. Pour renforcer le corps principal et lui donner une certaine rigidité, il est préférable de prévoir des renforts (9) sur la face externe du fond (2). On peut prévoir un renfort longitudinal (9') s'étendant d'une tubulure (10) à l'autre (11) et des renforts transversaux (9") s'étendant d'une partie de la paroi à l'autre. Ces renforts (9) s'étendent jusqu'au niveau du bord libre de la paroi (3). Plutôt que de prévoir un corps principal creux éventuellement renforcé, il aurait également été possible de le réaliser plein.

Dans une variante de réalisation non représentée, le renfort est constitué d'une seule nervure longitudinale (9') s'étendant d'une tubulure (10) à l'autre (11). Une lucarne est pratiquée dans cette nervure (9') pour permettre le blocage du récipient dans la machine d'hémodialyse. Ce blocage est obtenu avec un levier (13) de la machine qui vient s'encliqueter dans la lucarne et empêche la sortie involontaire du récipient et de l'insert hors de la machine. Pour assurer un bon maintien vertical du récipient, deux nervures transversales peuvent être prévues de part et d'autre de la lucarne, une de chaque côte de la nervure longitudinale. Elles évitent que le récipient ne se couche sur le côté.

L'insert est réalisé par exemple par moulage en polyéthylène ou polypropylène.

Bien que les exemples présentés aient deux ouvertures (4, 5), il est tout à fait possible que l'insert n'en contienne qu'une, ou au contraire plus de deux. Dans tous les cas, il faut que les ouvertures se trouvent au fond d'un creux de sorte que le produit solide restant tende à y retomber pour être toujours soumis au flux de liquide. Il est préférable que la face interne du fond ne présente aucun endroit plat pouvant former un plateau horizontal sur lequel pourrait s'accumuler du produit solide. La face interne du fond doit donc de préférence ne présenter que des parties inclinées vers la ou les ouvertures, les parties inclinées situées autour d'un premier trou et les parties inclinées située autour d'un deuxième trou se rencontrant en formant des cols ou des crêtes sur lesquels le produit solide ne peut pas s'accumuler.

Grâce à l'insert de l'invention, il est possible de réaliser des poches, des cartouches, ou tout autre récipient, sans recoins ou espaces morts situés hors du flux de liquide. Ainsi, tout le produit solide situé dans le récipient se trouve dans le flux et peut être solubilisé. Les deux ouvertures étant situées dans les points les plus bas du récipient, tout le liquide peut être aspiré. En comparaison avec une forme plate du fond de l'insert, telle qu'on la connaît par exemple de EP 2 005 934 A2, le positionnement du ou des trous dans des cavités du fond, notamment la forme en vague entre les deux trous et son affaissement centrale, permet un meilleurs glissement des matières solides et moins de perturbation du flux en raison de la disparition d'une grande partie des angles et des coins. De même lors de l'introduction du liquide de mise en solution, les matières solides qui se sont accumulées dans ces cavités sont entraînées par le liquide se qui favorise leur mise en solution.

## Revendications

1. Insert pour récipient devant contenir un produit concentré sous forme solide, comprenant une partie principale (1) formée d'un fond (2) se prolongeant sur le bord par une paroi (3) perpendiculaire au fond, une première ouverture (4) et une deuxième ouverture (5) étant réalisées dans le fond, **caractérisé en ce que** la face du fond (2) destinée à être située dans le récipient après montage, face dite interne, est concave au niveau de la première ouverture (4) de sorte que la première ouverture se trouve au fond d'un creux et **en ce que** la face interne du fond (2) est concave au niveau de la deuxième ouverture (5) de sorte que la deuxième ouverture se trouve au fond d'un creux.

2. Insert selon la revendication précédente, **caractérisé en ce qu'**un filtre poreux est introduit dans les ouvertures (4, 5) ou dans le prolongement (10, 11) des ouvertures du côté de la face externe du fond pour empêcher le produit solide de quitter le récipient.

3. Insert selon la revendication 1 ou 2, **caractérisé en ce que** la face interne du fond (2) forme une vague de sorte que chaque ouverture (4, 5) se trouve dans un creux (6, 7) séparé de l'autre par la crête (8) de la vague.

4. Insert selon la revendication précédente, **caractérisé en ce que** la section transversale de la vague présente un creux (16).

5. Insert selon l'une des revendications précédentes, **caractérisé en ce que** la paroi (3) s'étend du côté de la face interne du fond et/ou du côté de la face opposée, dite face externe.

6. Insert selon l'une des revendications précédentes, **caractérisé en ce que** des renforts (9) solidaires des parois (3) sont prévus sur l'une au moins des faces du fond (2), de préférence sur la face externe.

7. Insert selon l'une des revendications précédentes, **caractérisé en ce que** l'une au moins des ouvertures (4, 5) se prolonge du côté de la face externe par une tubulure (10, 11).

8. Insert selon la revendication 7, **caractérisé en ce que** les deux ouvertures (4, 5) se prolongent du côté de la face externe par une tubulure (10, 11) et **en ce qu'**un renfort (9") est fixé sur la face externe du fond (2) en se prolongeant jusqu'aux deux tubulures (10, 11), le renfort (9") étant de préférence muni de moyens de blocage destinés à coopérer avec des moyens (13) correspondant sur la machine de prélèvement et/ou de traverses perpendiculaires au renfort (9").

9. Insert selon l'une des revendications précédentes, **caractérisé en ce que** la partie principale a la forme d'un vaisseau dont chaque pointe se prolonge de préférence par une languette (15).

10. Récipient devant contenir un produit concentré sous forme solide ou liquide, constitué d'un contenant soudé dans sa partie inférieure à un insert selon l'une des revendications précédentes.

## Patentansprüche

1. Einsatz für einen Behälter für ein konzentriertes Produkt in Form eines Feststoffs, welcher einen Hauptteil (1) umfasst, der aus einem Boden (2) gebildet ist, der am Rand durch eine zum Boden senkrecht verlaufende Wand (3) verlängert wird, wobei eine erste Öffnung (4) und eine zweite Öffnung (5) in dem Boden vorgesehen sind, **dadurch gekennzeichnet, dass** die Seite des Bodens (2), die dazu bestimmt ist, nach Einbau in dem Behälter angeordnet zu sein, Innenseite genannt, auf Höhe der ersten Öffnung (4) konkav ist, so dass sich die erste Öffnung im Boden einer Mulde befindet, und dadurch, dass die Innenseite des Bodens (2) auf Höhe der zweiten Öffnung (5) konkav ist, so dass sich die zweite Öffnung im Boden einer Mulde befindet.

2. Einsatz gemäß dem vorausgegangenen Anspruch, **dadurch gekennzeichnet, dass** ein poröser Filter in die Öffnungen (4, 5) oder in die Verlängerung (10, 11) der Öffnungen auf der Seite der Außenseite des Bodens eingebracht wird, um zu verhindern, dass das feste Produkt den Behälter verlässt.

3. Einsatz gemäß Anspruch 1 oder 2, **dadurch gekennzeichnet, dass** die Innenseite des Bodens (2) eine Welle bildet, so dass sich jede Öffnung (4, 5) in einer Mulde (6, 7) befindet, die voneinander durch den Kamm (8) der Welle getrennt sind.

4. Einsatz gemäß dem vorausgegangenen Anspruch, **dadurch gekennzeichnet, dass** der Querschnitt der Welle eine Mulde (16) aufweist.

5. Einsatz gemäß einem der vorausgegangenen Ansprüche, **dadurch gekennzeichnet, dass** sich die Wand (3) auf der Seite der Innenseite des Bodens und/oder auf der Seite der gegenüberliegenden Seite, Außenseite genannt, erstreckt.

6. Einsatz gemäß einem der vorausgegangenen Ansprüche, **dadurch gekennzeichnet, dass** mit der Wand (3) verbundene Verstärkungen (9) wenigstens auf einer der Seiten des Bodens (2), vorzugsweise auf der Außenseite, vorgesehen sind.

7. Einsatz gemäß einem der vorausgegangenen Ansprüche, **dadurch gekennzeichnet, dass** wenigstens eine der Öffnungen (4, 5) auf der Seite der Außenseite durch einen Stutzen (10, 11) verlängert wird.

8. Einsatz gemäß Anspruch 7, **dadurch gekennzeichnet, dass** die beiden Öffnungen (4, 5) auf der Seite der Außenseite durch einen Stutzen (10, 11) verlängert werden, und dadurch, dass eine Verstärkung (9") auf der Außenseite des Bodens (2) befestigt ist, die bis zu den beiden Stutzen (10, 11) verlängert ist, wobei die Verstärkung (9") vorzugsweise mit Blockiermitteln ausgestattet ist, die dazu bestimmt sind, mit entsprechenden Mitteln (13) an der Entnahmemaschine zusammenzuwirken, und/oder mit zur Verstärkung (9") senkrechten Querstreben.

9. Einsatz gemäß einem der vorausgegangenen Ansprüche, **dadurch gekennzeichnet, dass** der Hauptteil die Form eines Schiffs aufweist, dessen Spitze jeweils vorzugsweise durch eine Zunge (15) verlängert wird.

10. Behälter für ein konzentriertes Produkt in Form eines Feststoffs oder einer Flüssigkeit, gebildet aus einem Behältnis, das an seinem unteren Teil an einen Einsatz gemäß einem der vorausgegangenen Ansprüche geschweißt ist.

## Claims

1. Insert for a receptacle that is to contain a concentrated product in solid form, comprising a main part (1) formed by a base (2) extended over the edge by a wall (3) perpendicular to the base, a first opening (4) and a second opening (5) being produced in the base, **characterised in that** the face of the base (2) intended to be situated in the receptacle after assembly, the so-called internal face, is concave at the first opening (4) so that the first opening is situated at the bottom of a trough, and **in that** the internal face of the base (2) is concave at the second opening (5) so that the second opening is situated at the bottom of a trough.

2. Insert according to the preceding claim, **characterised in that** a porous filter is introduced into the openings (4, 5) or in the extension (10, 12) of the openings on the external face of the base (2) in order to prevent the solid product from leaving the receptacle.

3. Insert according to claim 1 or 2, **characterised in that** the internal face of the base (2) forms a wave so that each opening (4, 5) is situated in a trough (6, 7) separated from the other by the crest (8) of the wave.

4. Insert according to the preceding claim, **characterised in that** the transverse section of the wave have a trough (16).

5. Insert according to one of the preceding claims, **characterised in that** the wall (3) extends on the same side as the internal face of the base and/or on the same side as the opposite face, referred to as the external face.

6. Insert according to one of the preceding claims, **characterised in that** reinforcements (9) fixed to the walls (3) are provided on at least one of the faces of the base (2), preferably on the external face.

7. Insert according to one of the preceding claims, **characterised in that** at least one of the openings (4, 5) is extended on the same side as the external face by a tube (10, 11).

8. Insert according to claim 7, **characterised in that** both of the openings (4, 5) are extended on the same side as the external face by a tube (10, 11) and that a reinforcement (9") is fixed to the external face of the bottom (2) while being extended as far as the two tubes (10, 11), the reinforcement (9") preferably being provided with locking means intended to cooperate with corresponding means (13) on the take-off machine and/or cross members perpendicular to the reinforcement (9").

9. Insert according to one of the preceding claims, **characterised in that** the main part is in the form of a vessel, each point of which is preferable extended by a tongue (15).

10. Receptacle that is to contain a concentrated product in solid or liquid form, consisting of a container welded in its bottom part to an insert according to one of the preceding claims.
